# EUROPEAN PATENT APPLICATION

(11) **EP 2 402 356 A2**
(43) Date of publication of application: **04.01.2012**
(21) Application number: 10172854.1
(22) Date of filing: 15.08.2010
(51) Int. Cl.: C07H 19/06, A61K 31/7052, A61P 31/18

(54) **Modified 2',3'-dideoxynucleosides for treatment infections caused by human immunodeficiency virus (HIV) multidrug resistant strains, method of their synthesis and the pharmaceutical agent containing these nucleosides**

(30) Priority: 30.06.2010 PL 39169410
(71) Applicant: Instytut Biochemii I Biofizyki Pan, 02-106 Warszawa (PL)
(72) Inventor: ZIEMKOWSKI, Przemyslaw, 03-130, Warszawa (PL); MIAZGA, Agnieszka, 37-400, Nisko (PL); KULIKOWSKI, Tadeusz, 02-116, Warszawa (PL); KLIMKAIT, Thomas, CH4051, Basel (CH); LOUVEL, Severine, CH4051, Basel (CH); HAMY, Francois, 68110 Illzach (FR); Lipniacki, Andrzej, 03-982, Warszawa (PL); PIASEK, Andrzej, 02-511, Warszawa (PL)
(74) Representative: Brodowska, Iwona

(57) **Abstract**

2',3'-Dideoxy-3'-fluoro-4-thiothymidine (4-SFLT) derivatives according to the invention substituted at 5'-O position of 4-SFLT with 12-tetradodecanoyl, 12-bromododecanoyl, 12-metoxydodecanoyl, 12-ethylothiododecanoyl, 11-ethylothioundecanoyl or 12-azidodocanoyl group (represented by the symbols **WA18, WA19, WA21,WA22,WA23** and **WA20** in the deCIPhR™ cell system exert high antiviral activity against wild type HIV-1 strain, as well as against its drug and multidrug resistant strains, and moreover very low citotoxicity (CC₅₀ > 200 µM) and very high selectivity.

The compounds, because of lack of toxicity may be applied at all AIDS phases i.e. also them the T4 lymphocytes level in patients drops down below 200/ µL of peripheral blood.

2',3'-Dideoxy-3'-fluoro-4-thiothymidine derivatives according to the invention are synthesized by the transformation of the known compound 2',3'-dideoxy-3'-fluoro-4-thiothymidine (4-SFLT).

## Description

### Technical Field

The presented invention relates to new modified 2',3'-dideoxynucleosides active against human immunodeficiency virus (HIV-1) including its drug and multidrug resistant strains, namely 2',3'-dideoxy-3'-fluoro-4'-thiothymidine derivatives represented by the general **Formula 1**, wherein **R** represents 12-tetradodecanoyl, 12-bromododecanoyl, 12-metoxydodecanoyl, 12-ethylothiododecanoyl, 11-ethylothioundecanoyl or 12-azidododecanoyl group, method of their synthesis and comprising them pharmaceutical agents.

New compounds are, according to the invention, pro-drugs of the selective inhibitor of HIV-1 retrovirus - 2',3'-dideoxy-3'-fluoro-4-thiotymidine (4-SFLT). The pro-drugs need intracellular hydrolytic cleavage yielding 4-SFLT followed by its intracellular activation - phosphorylation with host kinases yielding its nucleoside-5'-triphosphate.

### Background Art

According to current knowledge, six 5'-O-myristoyl 2',3'-dideoxy-3'-fluoro-thymidine derivatives are known so far, and they belong to the most active HIV inhibitors in various T cells, much more potent than the standard drug AZT. Unfortunately, FLT released in cells is more toxic towards non-infected lymphocytes than AZT (Balzarini J., Baba M., Pauwels R., Herdewijn P., De Clercq E. Biochem. Pharmacol. 1988, 37, 2847-2856). Clinical evaluations have shown that thrombocytopenia and anemia are the toxic effects which limit the drug dose (Balzarini J., Baba M., Pauwels R., Herdewijn P., De Clercq E. Biochem. Pharmacol. 1988, 37, 2847-2856; Kong X.-B., Zhu Q.-Y., Vidal P.M., Watanabe K.A., Polsky B., Armstrong D., Ostrander M., Lang S.A. Jr., Muchmore E., Chou T.-C. Antimicrob. Agents Chemother., 1992, 36, 808-818; Matthes E., Lehnmann C.M., Scholz D., Rosenthal H.A., Langen P. Biochem. Biophys. Res. Commun. 1988, 153, 825-831) and for this reason further clinical evaluations have been stopped. Above adverse side effects obliged the inventor to look for FLT analogs exerting lower toxicity among the analogues of 5'-O-substituted 4-thioderivatives class. The compounds of this type show, as compared to FLT, lower toxicity, higher lipophilicity and better penetration through the animal cells membranes and across blood-brain barrier. 2',3'-Dideoxy-3'-fluoro-4-thiothymidine (4-SFLT) was previously synthesized with the use of Lawesson reagent by thiation of sugar protected FLT in boiling 1,4-dioxane (Matthes et al., 1987, EP 0 254 268 AZ) but 4-SFLTMP has not been known compound. Because of very low toxicity, the compounds of proposed type may be applied at all phases of AIDS development, also in the final phase, when the amount of T₄ lymphocytes in peripheral blood of patients drops down below 200/µL.

The inventors unexpectedly discovered that according to the invention it is possible to obtain a new 5'-O-myristoylated 4-thio-FLT derivatives represented by the general **Formula 1** wherein **R** represents 12-tetradodecanoyl, 12-bromododecanoyl, 12-methoxydodecanoyl, 12-ethylothiodedecanoyl, 11-ethylothioundecanoyl or 12-azidododecanoyl group, by thiation of known compounds, 5'-O-acetylo-2',3'-dideoxy-3'-fluorothymidine (5'-OAcFLT) (Huang et al., J. Med. Chem., 1991, 34, 1640-1646) in boiling dry tetrahydrofurane (THF), so at significantly lower temperature than the temperature of boiling 1,4-dioxane. Deblocking of sugar (Matthes et al., 1987, EP 0 254 268 AZ) followed by esterification of 5'-OH group by treating with a respective fatty acid halogen leads to the compounds of pro-drug character exerting potent and selective antiretroviral (anti-HIV-1) activity.

The inventors unexpectedly discovered also that new 4-SFLT derivatives according to the invention possessing thio group at 4 position of FLT pyrimidine ring and 12-tetradodecanoyl, 12-bromododecanoyl, 12-methoxydodecanoyl, 12-ethylothiododecanoyl, 11-ethylothioundecanoyl or 12-azidododecanoyl group at 5'-O-position exert in deCIPhR™, CEMA and CEM-T4 cells very high antiviral activity against wild, drug and multidrug resistant HIV-1 strains **(Table 1)** and very high selectivity (cytotoxicity not measurable).

**Summary of invention**

New 2',3'-dideoxy-3'-fluoro-4-thio-thymidine analogues - the pro-drugs exerting high activity against human immunodeficiency virus (HIV-1) represented by the general **Formula 1,** wherein **R** represents 12-tetradodecanoyl, 12-bromododecanoyl, 12-methoxydodecanoyl, 12-ethylothiododecanoyl, 11-ethylothioundodecanoyl or 12-azidododecanoyl group represented by the symbols **WA18, WA19, WA21, WA22, WA23** and **WA20** respectively.

Particularly favorable 4-SFLT derivatives are compounds represented by the **Formula 1,** wherein **R** represents 12-tetradodecanoyl, 12-bromododecanoyl and 12-methoxydodecanoyl group, that means the compounds represented by the symbols **WA18, WA19** and **WA21** respectively.

The method of synthesis of 5'-O-myristoylated 4-SFLT derivatives represented by the general **Formula 1** wherein **R** represents 12-tetradodecanoyl, 12-bromododecanoyl, 12-methoxydodecanoyl, 12-ethylothiododecanoyl, 11-ethylothioundecanoyl or 12-azidododecanoyl groups represented by the symbols **WA18, WA19, WA21, WA22, WA23** and **WA20** respectively, according to the invention comprise:

(a) the compounds represented by the **Formula 1** and the symbols **WA18, WA19, WA21, WA22** and **WA23,** wherein **R** represents the fatty acid group of 12-tetradodecanoyl, 12-bromododecanoyl, 12-methoxydodecanoyl, 12-ethylothiododecanoyl, or 11-ethylothioundecanoyl acid are obtained by treating the compound 4-SFLT (**WA2, Formula 1,** wherein **R** represents hydrogen) in dichloromethane in the presence of dimethyloaminopyridine for one hour at room temperature with a respective halogen acid of formula **RX** wherein **R** represents as above and **X** represents halogen ( **Scheme I**);

(b) the compound represented by the **Formula 1** and the symbol **WA20** wherein **R** represents 12-azidododecanoyl group is obtained by treating the compound represented by the **Formula 1** (wherein **R** represents 12-bromododecanoyl group) and the symbol **WA19,** with sodium azide in dimethyloformamide at room temperature **(Scheme II).**

Pharmaceutical agent with 4-SFLT **(WA2)** pro-drug character exerting a high activity against human immunodeficiency virus (HIV-1) containing known, pharmaceutically acceptable media and auxiliary materials which according to the invention comprises as an active component the compound represented by the general **Formula 1,** wherein **R** represents 12-tetradodecanoyl, 12-bromododecanoyl, 12-methoxydodecanoyl, 12-ethylothiododecanoyl, 11-ethylothioundecanoyl or 12-azidododecanoyl group, which is hydrolyzed in a cell by esterases yielding 4-SFLT represented by the general **Formula 1** and the symbol **WA2** or contains a mixture of the compounds represented by the **Formula 1** and the symbols **WA18, W19, WA20, WA21, WA22** or **WA23.**

Favorably pharmaceutical agent, according to the invention, contains the compounds represented by the symbols **WA18, WA19** and/or **WA22,** also as their mixture.

Pharmaceutical agent containing compounds represented by the symbols **WA18, W19, WA20, WA21, WA22** and **WA23,** according to the invention exerts a potent inhibition of wild type, drug and multidrug resistant (HIV-1) strains in deCIPhR™, CEMA and CEM-T4 cells **(Table 1)** as well as potent inhibition of the viruses resistant to the so far known drugs, without any cytotoxicity at the therapeutic concentration (CC₅₀ > 200 µM).

The examples are presented below which illustrate the invention and do not limit its scope.

Description of embodiments

**Example I.**

**General method for the synthesis of 5'-O-myristoylated derivatives of 2',3'-dideoxy-3'-fluoro-4-thiothymidine**

To the solution of the appropriate fatty acid (2 mmol) in dry dichloromethane (40 ml) oxalyl chloride (0.4 mL, 4.2 mmol) was added and the mixture was stirred 2 h at room temperature and evaporated to dryness *in vacuo.* Dry residual oil was dissolved in 20 mL of dry dichloromethane and the solution was added dropwise to an ice-cold solution of 4-SFLT (520 mg, 2 mmol) and 4-dimethyloaminopyridine (DMAP) (352 mg, 2.8 mmol) in dry dichloromethane (40 mL). This solution was stirred at 0°C for 20 min and then at room temperature for 2 h. The mixture was washed with saturated aqueous sodium bicarbonate (2 × 50 mL) and with water (2 × 50 mL). The organic layer was dried with Na₂SO₄ and the solvent was removed *in vacuo.* The residue was purified by silica gel chromatography using chloroform as eluent to yield the respective products **WA19, WA21, WA22, WA23.**

1-(5-O-(12-bromododecanoyl)-2.3-dideoxy-3-fluoro-b-D-ribofuranosyl)-4-th iothymine **(WA19)**

Yield: 0.96 g, 92%; ¹H NMR (CDCl₃) 9.47 (brs, 1 H, NH), 7.32 (d, 1 H, H6), 6.27 (dd, 1H, H1'), 5.26-5.11 (dd, 1 H, H3', J_{3',F}= 53.32 Hz), 4.55-4.47 (dt, 1 H, H4', J_{4',F}=25.54), 4.40 and 4.28 (two dd, 1 H each, H5', H5"), 3.40 (t, 2H, CH₂Br), 2.81-2.71 (m, 1 H, H2"), 2.33 (t, 2H, CH₂CO), 2.1 (d, 3H, 5-CH 3), 2.16-2.0 (m, 1 H, H-2'), 1.85 (quintet, 2H, CH₂CH₂Br), 1.65-1.58 (m, 2H; CH₂CH₂CO), 1.43-1.27 (br m; 14H, (CH₂)₇); MS (M+H)⁺ 521.

1-(2,3-dideoxy-3-fluoro-5-O-(12-methoxydodecanoyl)-b-D-ribofuranosyl)-4-thiothymine **(WA21)**

Yield 0.58 g, 62%; ¹H NMR (CDCl₃) 9.55 (brs, 1 H, NH), 7.32 (d, 1 H, H6), 6.27 (dd, 1H, H1'), 5.26-5.11 (dd, 1 H, H3', J_{3',F}= 53.32 Hz), 4.55-4.47 (dt, 1 H, H4', J_{4',F}=25.54), 4.40 and 4.28 (two dd, 1 H each, H5', H5"), 3.36 (t, 2H, CH₂OMe), 3.33 (s, 3H, OCH₃), 2.82-2.71 (m, 1 H, H2"), 2.33 (t, 2H, CH 2CO), 2.17 (d, 3H, 5-CH₃), 2.18-1.98 (m, 1 H, H-2'), 1.65-1.52 (m, 4H; CH₂ CH₂OMe i CH₂CH₂CO), 1.32-1.25 (br m; 14H, (CH₂)₇); MS (M+H)⁺ 473.

1-(2,3-dideoxy-5-O-(12-thioethyldodecanoyl)-3-fluoro-b-D-ribofuranosyl)-4-thiothymine **(WA22)**

Yield 0.84 g, 84%; 1 H NMR (CDCl₃) 9.44 (s, 1 H, NH), 7.32 (d, 1 H, H6), 6.27 (dd, 1H, H1'), 5.26-5.11 (dd, 1 H, H3', J_{3',F}= 53.32 Hz), 4.55-4.47 (dt, 1 H, H4', J_{4',F}=25.54), 4.40 and 4.38 (two dd, 1 H each, H5', H5"), 2.81-2.71 (m, 1 H, H-2"), 2.48-2.59 (m, 4H, CH₂CH₂S, CH₃CH₂S), 2.33 (t, 2H, CH₂ CO), 2.1 (d, 3H, 5-CH₃), 2.16-2.0 (m, 1 H, H-2'), 1.65-1.53 (m, 4H, CH₂CH₂ S, CH₂CH₂CO), 1.38-1.2 (br m, 17H, (CH₂)₇ i CH₃); MS (M+H)⁺ 503.

1-(5-O-(2,3-dideoxy-11-thioethylundecanoyl)-3-fluoro-b-D-ribofuranosyl)-4-thiothymine **(WA23)**

Yield 0.86 g, 88%; ¹H NMR (CDCl₃) 9.44 (brs, 1 H, NH), 7.32 (d, 1 H, H6), 6.27 (dd, 1H, H1'), 5.26-5.11 (dd, 1 H, H3', J_{3',F}= 53.32 Hz), 4.55-4.47 (dt, 1 H, H4', J_{4',F}=25.54), 4.40 and 4.28 (two dd, 1 H each, H5', H5"), 2.81-2.71 (m, 1 H, H2"), 2.56-2.49 (m, 4H, CH₂CH₂S, CH₃CH₂S), 2.33 (t, 2H, CH₂ CO), 2.1 (d, 3H, 5-CH₃), 2.16-2.0 (m, 1 H, H-2'), 1.66-1.53 (m, 4H; CH₂CH₂ S i CH₂CH₂CO),1.38-1.25 (br m, 12H, (CH₂)₆), 1.25 (t, 3H, CH₃) MS (M+H)⁺ 489.

**Example II.**

1-(2,3-dideoxy-3-fluoro-5-O-(12-tetradodecanoyl)-b-D-ribofuranosyl)-4-thio thymine **(WA18)**

Myristoyl chloride (0.54 mL, 2 mmol) was added dropwise to the ico-cold mixture of 4-SFLT (520 mg, 2 mmol) and 4-dimethylaminopyridine (352 mg, 2.8 mmol) in dry dichloromethane (40 mL). This solution was stirred at 0°C for 1 h, and then at room temperature for 2 h. The mixture was washed with saturated aqueous sodium bicarbonate (2 x 50 mL) and with water (2 x 50 mL). The organic layer was dried with Na₂SO₄ and the solvent was removed *in vacuo.* The residue was purified by silica gel chromatography using using chloroform as eluent to yield compound WA18 (0,55 g, 59%); ¹ H NMR (CDCl₃) 9.41 (s, 1 H, NH), 7.32 (d, 1 H, H6), 6.27 (dd, 1 H, H1'), 5.26-5.11 (dd, 1 H, H3', J_{3',F}= 53.32 Hz), 4.55-4.47 (dt, 1 H, H4', J_{4',F} =25.54), 4.40 and 4.38 (two dd, 1 H each, H5', H5"), 2.81-2.71 (ddd, 1 H, H-2"), 2.33 (t, 2H, CH₂CO), 2.16-1.97 (m, 1 H, H2'), 2.1 (d, 3H, 5-CH₃), 1.66-1.55 (m, 2H, CH₂CH₂CO), 1.30-1.25 (br m, 20H, (CH₂)₁₀), 0.87 (t, 3H, CH₃); MS (M+H)⁺ 471.

**Example III.**

1-(5-O-(12-azidododecanoyl)-2,3-dideoxy-3-fluoro-b-D-ribofuranosyl)-4-thi othymine **(WA20)**

The solution of 1-(5-O-(12-bromododecanoyl)-2,3-dideoxy-3-fluoro-4-thiothymidine (520 mg, 1 mmol) and sodium azide (97.5 mg, 1.5 mmol) in dimethylformamide (10 mL) were stirred for 3 h at room temperature. The solvent was then removed *in vacuo* and the residue was dissolved in chloroform (40 mL) and extracted with water (2 x 40mL). The organic layer was dried over Na₂ SO₄ and evaporated to dryness *in vacuo.* The oil residue was purified by silica gel chromatography using chloroform as eluent to yield compound **WA20** (0,47 g, 90%); ¹H NMR (CDCl₃) 9.39 (s, 1 H, NH), 7.32 (d, 1 H, H6), 6.27 (dd, 1H, H1'), 5.26-5.11 (dd, 1 H, H3', J_{3',F}= 53.32 Hz), 4.55-4.47 (dt, 1 H, H4', J_{4',F}=25.99), 4.40 and 4.38 (two dd, 1 H each, H5', H5"), 3.25 (t, 2H, CH₂N₃), 2.81-2.71 (m, 1 H, H2"), 2.33 (t, 2H, CH₂CO), 2.16-2.00 (m, 1 H, H2'), 2.1 (d, 3H, 5-CH3), 1.64-1.56 (m, 4H; CH₂CH₂CO, CH₂CH₂N₃), 1.37-1.26 (br m, 14H, (CH₂)₇); MS (M+H)⁺ 484.

**Example IV.**

The antiviral activity (EC₅₀) and cytotoxicity (CC₅₀) of the derivatives obtained as above (Examples I-III) were investigated in the deCIPhR™ cell system with use of the following methods.

*Determination of antiviral activity*

Fully infectious virus HIV-1 was produced from cloned proviral DNA reference coding either for wild-type HIV-1 or from drug-resistant virus following transfection of proviral DNA into recipient mammalian cells. Subsequent to viral protein expression and particle formation, culture supernatant was harvested and used to infect an indicator cell line. This latter cell line expresses all necessary receptors and co-receptors for HIV infection as well as the bacterial gene LacZ coding for β-galactosidase under the control of the HIV-1 long terminal repeat. In this system, amount of β-galactosidase is directly proportional to the extent of viral replication. Activity of β-galactosidase is assayed in presence of a colorless substrate which upon enzymatic conversion yields a yellow product absorbing at 405 nm. Next, variation in colorimetric readout in presence of test compounds was translated in percent viral inhibition following normalization according to absorbance values of positive (presence of anti-HIV drug) and negative (untreated) control wells included in each 96 well-plate. For determining effective concentration 50 % (EC₅₀), test compounds were assayed across a range of concentrations. Percent inhibition data in function of drug concentration were processed by a statistical curve fitting software yielding a modeled curve from which EC₅₀ was extrapolated.

*Determination of cytotoxicity. Alamar Blue* ™ *Cell Viability Assay*

The Alamar Blue™ Cell Viability Assay (Invitrogen) is a fluorimetric metod for determining the number of viable cells. The Alamar Blue Reagent contains the cell permeable non-fluorescent blue dye resazurin, which is converted to the pink and fluorescent dye resorufin in response to the action of reductases located in the cytosol, mitochondria and endoplasmic reticulum. Therefore, only living cells will convert resazurin into resorufin. Assays are performed by adding 20 µL of the Alamar Blue Reagent directly to culture wells followed by a 6-hour incubation at 37°C. The fluorescent signal is monitored using 545 nm excitation wavelength and 590 nm emission wavelength. The quantity of resofurin product as measured by fluorescence is directly proportional to the number of living cells in culture, what allows to calculate CD₅₀ value. In this case CD₅₀ values were not determined because of their very low level (compounds **18-23** are not toxic).

**Table 1.** Antiviral activity (EC₅₀) of thiated at position 4, 5'-O-mirystoylated derivatives of FLT in deCIPhR™^{c}, CEMA^{a,c} and CEM-T4^{b,c} cells.

**Table 1**

| Szczep HIV-1 | EC₅₀ µM | | | | | | | | |
|---|---|---|---|---|---|---|---|---|---|
| | WA- 18 | WA- 19 | WA- 2 0 | WA2 1 | WA-2 2 | WA-2 3 | FLT MP | 4-SFL TMP | AZTM P² |
| Wild type pNL4-3 | 0.421 | 0.71 | 0.822 | 0.269 | 0.998 | 0.965 | 0.010 | 0.097 | 0.063 |
| Recombin ant provirus from the patient 6021959³ | 8.343 | 10.0 | 4.637 | 9.259 | 10.00 | 5.518 | 0.030 | 0.338 | 1.712 |
| Recombin ant provirus from the patient 7007984⁴ | 2.402 | 6.03 9 | 2.878 | 3.151 | 5.924 | 10.00 0 | 0.018 | 0.425 | 1.027 |
| Wild type #^{a,b} | 0.130 | 0.09 0 | 0.450 | 0.330 | 0.120 | 0.450 | ≤0.01 2 | <0.030 | ≤0.01 2 |
| NNRTI (RTMDR) 5 resistant | 0.045 | 0.02 7 | 0.100 | 0.550 | ≤0.05 0 | 0.480 | ≤0.01 2 | 0.020 | ≤0.01 2 |
| NRTI (RTMC)⁶ resistant | 0.150 | 0.22 0 | ≤100 | 0.180 | 0.075 | 0.450 | ≤0.01 2 | 0.100 | N/D |

¹F. Hamy et al., InPheno Study Report # 0430-8

²Reference compound

³HIV-1 reverse transcriptase (RT) resistance mutations: M41 L, E44D, T69D, L741, K103N, V108I, V118I, M184V, M230L, T215Y, G333E

⁴HIV-1 reverse transcriptase (RT) resistant mutations: M41 L, K70R, A985, T215Y

⁵ non-nucleoside HIV reverse transcriptase inhibitors

⁶ nucleoside HIV reverse transcriptase inhibitors

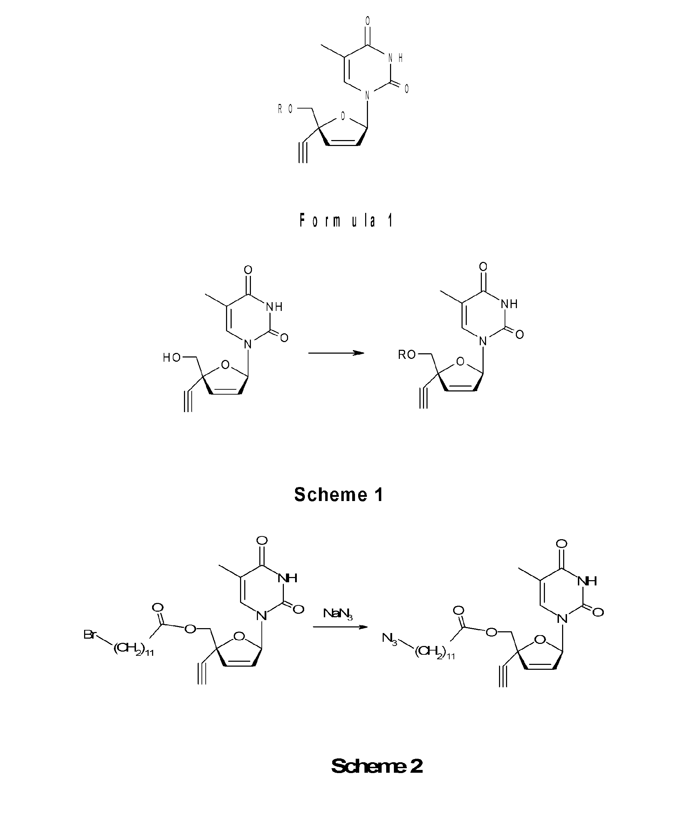

## Claims

1. Modified 2',3'-dideoxynucleosides for treatment infections caused by human immunodeficiency virus (HIV) multidrug resistant strains represented by the general **Formula 1,** wherein **R** represents 12-tetradodecanoyl, 12-bromododecanoyl, 12-metoxydodecanoyl, 11-ethylothioundecanoyl or 12-azidododecanoyl group and by the symbols **WA18, WA19, WA21, WA23** and **WA20** respectively.

2. The derivatives according to the claim 1 represented by the general **Formula 1,** wherein **R** represents for the compound **WA18** 12-tetradodecanoyl group, for the compound **WA19** 12-bromododecanoyl, for the compound **WA21** 12-metoxydodecanoyl, for the compound **WA23** 11-ethylothioundecanoyl or for the compound **WA20** 12-azidododecanoyl group.

3. The derivatives according to the claim 1 represented by the general **Formula 1,** wherein **R** represents 12-tetradodecanoyl, 12-bromododecanoyl, 12-metoxydodecanoyl, 11-ethylothioundecanoyl, 12-ethylothiododecanoyl, or 12-azidododecanoyl group.

4. The method of synthesis of 5'-O-substituted myristoylated 2',3'-dideoxy-3'-fluoro-4-thiothymidine derivatives represented by the general **Formula 1,** wherein **R** represents 12-tetradodecanoyl, 12-bromododecanoyl, 12-metoxydodecanoyl, and 11-ethylothioundecanoyl group represented by the symbols **WA18, WA19, WA21,** and **WA23** respectively, comprises treating the compound represented by the symbol **WA2** (4-SFLT) in dichloromethane in the presence dimethyloaminopyridine for one hour with a respective fatty acid halogene represented by the formula **RX,** wherein **R** represents as above, and **X** represents halogen **(Scheme I).**

5. The method of synthesis of 5'-O-substituted myristoylated 2',3'-dideoxy-3'-fluoro-4-thiothymidine derivative represented by the general **Formula 1,** wherein **R** represents12-azidododecanoyl group comprises treating the compound represented by the general **Formula 1,** wherein **R** represents 12-bromododecanoyl group **(WA19)** with sodium azide in dimethyloformamide at room temperature **(Scheme II).**

6. Pharmaceutically acceptable form of drug containing known, pharmaceutically acceptable media and auxiliary substances, containing as an active component the derivatives represented by the general **Formula 1,** wherein **R** represents 12-tetradodecanoyl, 12-bromododecanoyl, 12-metoxydodecanoyl, 11-ethylothioundecanoyl, and/or 12-azidododecanoyl group according to the claim 1.

7. The form according to the claim 6 containing as an active component a mixture of whichever of the compounds represented by the general **Formula 1,** wherein **R** represents 12-tetradodecanoyl, 12-bromododecanoyl, 12-metoxydodecanoyl, 11-ethylothioundecanoyl and 12-azidodocanoyl group (compounds represented by the symbols **WA18, WA19, WA21,WA23** and **WA20** respectively.
